# EUROPEAN PATENT SPECIFICATION

(11) **EP 1 761 241 B1**
(45) Date of publication and mention of the grant of the patent: **02.07.2008**
(21) Application number: 05746730.0
(22) Date of filing: 26.04.2005
(51) Int. Cl.: A61K 8/73, A61K 8/81, A61Q 15/00

(54) **USE AS AN ANTIPERSPIRANT AGENT OF A FLOCCULATING WATER-SOLUBLE POLYMER; COSMETIC PROCESS FOR TREATING PERSPIRATION**
VERWENDUNG EINES WASSERLÖSLICHEN FLOCKUNGSPOLYMERS ALS ANTITRANSPIRANT; KOSMETISCHES VERFAHREN ZUR BEHANDLUNG VON SCHWEISSABSONDERUNG
UTILISATION D'UN POLYMERE HYDROSOLUBLE FLOCULANT EN TANT QU'AGENT ANTISUDORAL, ET PROCEDE COSMETIQUE DE TRAITEMENT DE LA TRANSPIRATION

(30) Priority: 09.06.2004 FR 0451140; 13.07.2004 US 587041 P
(43) Date of publication of application: 14.03.2007
(73) Proprietor: l'Oreal SA, 75008 Paris (FR)
(72) Inventor: LEMOINE, Cyril, F-95380 Puiseux-en-France (FR); BEAU, Nathalie, F-95610 Eragny-sur-Oise (FR)
(86) International application number: PCT/EP2005/005243
(87) International publication number: WO 2005/120448

(56) References cited:
- EP-A- 0 222 580
- EP-A- 0 478 327
- US-A- 4 675 178
- US-A- 4 690 817
- US-A1- 2002 119 108

## Description

The present invention relates to the use of a particular flocculating water-soluble polymer comprising amine groups borne by a side substituent directly attached to the main chain, as an antiperspirant active agent in a cosmetic composition and particularly in a cosmetic composition not containing any antiperspirant aluminium and/or zirconium salts.

The present invention also relates to a cosmetic process for treating perspiration, which consists in applying to the surface of the skin an effective amount of a flocculating water-soluble polymer comprising amine groups borne by a side substituent directly attached to the main chain.

It is well known in cosmetics to use in topical application antiperspirant products containing substances that have the effect of limiting or even suppressing the flow of sweat. These products are generally available in the form of roll-ons, sticks, aerosols or sprays.

Antiperspirant substances generally consist of aluminium salts or aluminium/zirconium salts. Their antiperspirant efficacy is limited when they are used alone. The use of these active agents at high concentrations to obtain good efficacy results in formulation difficulties. Furthermore, these substances have an irritant potential to the skin.

There is thus a need to find novel antiperspirant active agents that can replace aluminium salts and aluminium/zirconium salts, and that are effective and easy to formulate.

Water-insoluble polymers forming an occlusive film on the skin have already been proposed in patent application WO 95/24105 as antiperspirant active agents. It is not necessary to use standard aluminium salts. The occlusive polymers proposed are of the octacrylamide/acrylates copolymer type or of the vinyl acetate/butyl maleate/isobornyl acrylates copolymer type, alone or in combination with a PVP/linear α-olefin polymer, for instance PVP/eicosene.

Water-insoluble film-forming polymers whose main chain is hydrocarbon-based and which comprise pendent hydrophobic quaternary ammonium groups have also been proposed in patent application WO 95/27473 as antiperspirant active agents.

Patent application WO 01/54658 discloses anhydrous compositions containing a cyanoacrylate monomer that reacts with sweat to form in situ by polymerization a film on the skin that blocks the sweat ducts.

However, these occlusive film-forming polymers do not make it possible to obtain fully satisfactory antiperspirant efficacy and still elicit formulation problems.

Moisture-absorbing polymers have been proposed as substitutes for standard astringent salts in antiperspirant compositions in patent US 4 743 440. These moisture-absorbing polymers may especially be water-soluble and chosen especially from natural gums (xanthan, agar, carrageenans, guar or gelatin), celluloses (hydroxypropylmethylcellulose, carboxymethylcellulose), polyoxyethylenes, polyvinylpyrrolidones, polycarboxyvinylics or vinyl ether/maleic anhydride copolymers. In patent application WO 03/030 853, the recommended moisture-absorbing polymers are chosen from grafted starch homopolymers and copolymers of 2-propenamide-co-propenoic acid sodium salt.

However, these moisture-absorbing polymers do not make it possible to obtain fully satisfactory antiperspirant efficacy and still elicit formulation problems.

Water-soluble quaternary polymers have been proposed in antiperspirant compositions in the presence of standard aluminium salts to improve their efficacy. This is the case for dimethyldiallylammonium chloride in patent application EP 222 580, which acts as an agent for retaining the antiperspirant salt. This is the case for the water-soluble polymers comprising a Brönsted acid in patent application WO 02/49590, in particular those derived from maleic acid and/or maleic anhydride, which act as co-gelling agent with the antiperspirant salts. This is the case for polyethyleneimines (PEI) in the article Cosmetics & Toiletries Vol. 108 August 1993 pages 73-77, which act as complexing agents for the aluminium salts.

Dimethyldiallylammonium chloride/acrylic acid copolymers have been proposed in patent application EP 478 327 as thickeners in aqueous liquid antiperspirant products containing aluminium salts.

In patent US 4 690 817, film-forming polymers of vinyl alcohols containing pendent quaternary amine groups have been proposed in antiperspirant compositions in the presence of standard astringent salts as skin conditioners, forming a moisturizing barrier thereon.

In patent application WO 82/01993, polyethyleneimines have been used as odour absorbers in particular of fatty acids, aldehydes or ketones and more particularly in alcohol-based deodorant products in spray or roll-on form. The Applicant has discovered, surprisingly, that particular flocculating water-soluble cationic polymers comprising amine groups borne by a side substituent directly attached to the main chain constitute by themselves excellent antiperspirants and can be easily formulated in numerous products for treating perspiration and perspiration-related body odour, without it being necessary to use standard astringent salts. One subject of the present invention is the use of a flocculating water-soluble polymer comprising amine groups borne by a side substituent directly attached to the main chain as defined in claim 1 as an antiperspirant active agent in a cosmetic composition and particularly in a cosmetic composition not containing any antiperspirant aluminium and/or zirconium salts. A subject of the present invention is also a cosmetic process for treating perspiration, which consists in applying to the surface of the skin an effective amount of a flocculating water-soluble polymer comprising amine groups borne by a side substituent directly attached to the main chain as defined in claim 1.

The term "antiperspirant agent" means any substance which, by itself, has the effect of reducing or limiting the flow of sweat without it being necessary to use an aluminium and/or zirconium antiperspirant salt.

The term "water-soluble polymer" means polymers which, when introduced into an aqueous phase at 25°C, at a mass concentration equal to 1%, make it possible to obtain a macroscopically homogeneous and transparent solution, i.e. a solution that has a minimum light transmittance value, at a wavelength equal to 500 nm, through a sample 1 cm thick, of at least 80% and preferably of at least 90%.

The term "flocculant" means any substance capable of destabilizing a colloidal suspension indistinctly via a flocculation or coagulation mechanism. The term "destabilization of colloids" means the formation of aggregates that make the suspension unstable. Since the terms flocculation and coagulation are generally interchangeable and equivalent, we will use the term "flocculation" in the invention to refer to either mechanism. The definitions of these mechanisms are given in Volume 10 of "Encyclopedia of Chemical Technology; Kirk-Othmer; 3rd edition".

From an experimental point of view, according to the invention, a flocculant will be considered as effective in antiperspirant terms if it reduces by at least 10% and better still 20% the transmittance measured at a wavelength of 700 nm of a solution of natural sweat filtered through a 200 micron filter.

The term "composition not containing any antiperspirant aluminium and/or zirconium salts" means any composition containing not more than 1% by weight of antiperspirant aluminium and/or zirconium salt.

The flocculating water-soluble polymers of the invention may be in the form of homopolymer, copolymer or terpolymer containing at least one monomer of quaternary ammonium type or a monomer of acrylamide type.

According to the invention, the term "polymer containing a monomer of quaternary ammonium type" means any polymer for which at least one amine derived from the side substituents is in the form of a quaternary amine between pH 4 and pH 8.

A nonionic spacer may optionally be introduced into these polymers, such as the following groups: alkylacrylates; alkylmethacrylates; vinyllactams such as vinylcaprolactone; vinylpyrrolidone; vinyl esters; vinyl alcohols; alkylene glycol, for instance propylene glycol or ethylene glycol.

The molar masses of these polymers generally range from 1000 to 20 000 000 g/mol, and they preferably have a molar mass ranging from 10 000 to 1 000 000 g/mol.

The flocculating water-soluble polymers comprising quaternary ammonium or amide groups that are pendent relative to the main chain, which are used in the compositions of the invention, are selected from : 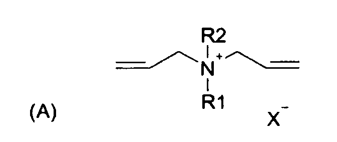
(i) diallyldimethylammonium chloride (DADMAC) homopolymers with a molar mass ranging from 10 000 to 1 000 000 g/mol, for instance Polyquaternium-6 (INCI name) of structure: 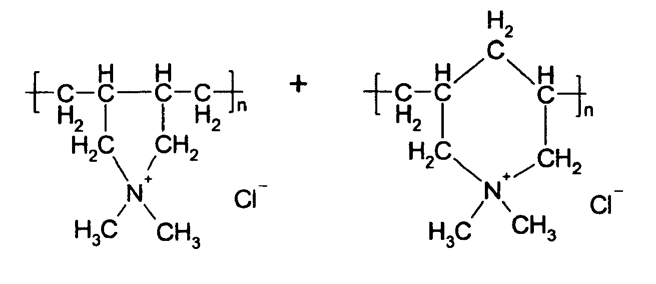
   Commercial products that may be mentioned include: Merquat 100^{®} (Ondeo Nalco): molar mass: 150 000 g/mol,Flocare C106^{®}: (SNF): molar mass: 400 000 g/mol, and Floquat FL45^{®} (SNF).
(ii) acrylamide/diallyldimethylammonium chloride copolymers or Polyquaternium-7 (INCI name) of structure: 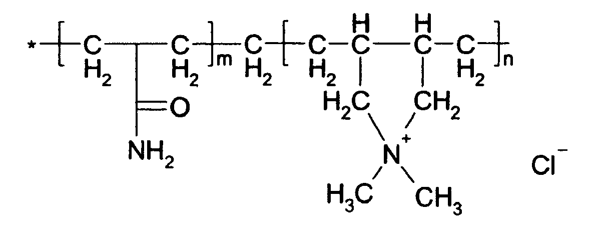
   Commercial products that may be mentioned include:
   Merquat 550 L (Ondeo-Nalco): m/n ratio = 50/50; molar mass: 2 000 000 g/mol.
   Salcare Super 7 (Ciba): m/n ratio = 75/25; molar mass: 100 000 g/mol.
   Flocare C 107^{®} (SNF): molar mass: 700 000 g/mol.
   Floerger D6000 Series^{®}: Floerger D 6030^{®} (SNF);
   Floerger D 6080^{®} (SNF); Floerger WS Series^{®} (SNF).
(iii) quaternary ammoniums of hydroxycellulose or Polyquaternium-10 that have reacted with an epoxide substituted with a trimethylammonium.
(iv) hydroxyalkyl celluloses, for instance hydroxymethyl-, hydroxyethyl- or hydroxypropyl celluloses grafted with a dimethyldiallylammonium salt. The marketed products are more particularly the products sold under the name Celquat L200^{®} and Celquat H100^{®} by the company National Starch.

The flocculating water-soluble polymers used as antiperspirant active agents are preferably present in the compositions according to the invention in amounts ranging from 0.1% to 50% and more preferably from 1% to 20% by weight relative to the total weight of the composition.

The compositions according to the invention intended for cosmetic use may be in the form of lotions, creams or fluid gels distributed as an aerosol spray, in a pump-dispenser bottle or as a roll-on, in the form of thick creams distributed in tubes or a grille; in the form of wands (sticks), and may contain in this regard the ingredients generally used in products of this type and well known to those skilled in the art.

The compositions according to the present invention intended for cosmetic use may comprise at least one aqueous phase. They are especially formulated as aqueous lotions or as water-in-oil, oil-in-water emulsions, or as multiple emulsions (oil-in-water-in-oil or water-in-oil-in-water triple emulsions (such emulsions are known and described, for example, by C. Fox in "Cosmetics and Toiletries", November 1986, Vol. 101, pages 101-112)).

The aqueous phase of the said compositions contains water and generally other water-soluble or water-miscible solvents. The water-soluble or water-miscible solvents include short-chain monoalcohols, for example of C₁-C₄, for instance ethanol or isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol. Propylene glycol and glycerol will more particularly be used.

According to one particular form of the invention, the antiperspirant compositions may be anhydrous.

For the purposes of the invention, the term "anhydrous" refers to a composition whose content of free or added water is less than 3% and preferably whose content of added water is less than 1% by weight relative to the total weight of the composition.

The compositions according to the invention preferably comprise at least one water-immiscible organic liquid phase. This phase generally comprises one or more hydrophobic compounds that render the said phase water-immiscible. The said phase is liquid (in the absence of a structuring agent) at room temperature (20-25°C). Preferentially the water-immiscible organic liquid phase in accordance with the invention generally consists of an oil or a mixture of oils and comprises at least 80% of compounds with a vapour pressure not exceeding 4 kPa (30 mmHg) at 25°C.

The water-immiscible organic liquid phase preferably contains one or more volatile or non-volatile, silicone-based or hydrocarbon-based emollient oils. These emollient oils are especially described in patents US 4 822 596 and US 4 904 463.

Volatile silicones are defined, in a known manner, as being compounds that are volatile at room temperature. Mention may be made among these compounds of cyclic and linear volatile silicones of the dimethylsiloxane type whose chains comprise from 3 to 9 silicone-based residues. Cyclomethicones D₄, D₅ or D₆ are preferably chosen.

Non-volatile silicones are defined, in a known manner, as being compounds with a low vapour pressure at room temperature. The following are included among these compounds: polyalkylsiloxanes, in particular linear polyalkylsiloxanes, for instance the linear polydimethylsiloxanes, or dimethicones, sold by the company Dow Corning under the name "Dow Corning 245 Fluid"; polyalkylarylsiloxanes, for instance the polymethylphenylsiloxanes sold by the company Dow Corning under the name "Dow Corning 556 Fluid"; copolymers of polyether and siloxane, for instance dimethicone copolyols.

Among the non-volatile emollient oils that may be used in the present invention, examples that may be mentioned include: hydrocarbon-based derivatives, mineral oils, fatty alcohols, esters of C₃-C₁₈ alcohols with C₃-C₁₈ acids, esters of benzoic acid with C₁₂-C₁₈ alcohols and mixtures thereof, C₂-C₆ polyols preferably chosen from glycerol, propylene glycol or sorbitol, polyalkylene glycol polymers.

The emollient oils are preferably present in amounts ranging from 1% to 50% by weight and more preferably from 5% to 40% by weight relative to the total weight of the composition.

The cosmetic compositions according to the invention may contain one or more additional deodorant active agents, for instance
- bacteriostatic agents or bactericidal agents such as 2,4,4'-trichloro-2'-hydroxydiphenyl ether (Triclosan), 2,4-dichloro-2'-hydroxydiphenyl ether, 3',4',5'-trichlorosalicylanilide, 1-(3',4'-dichlorophenyl)-3-(4'-chlorophenyl)urea (Triclocarban) or 3,7,11-trimethyldodeca-2,5,10-trienol (Farnesol); quaternary ammonium salts, for instance cetyltrimethylammonium salts or cetylpyridinium salts; chlorhexidine and salts; diglyceryl monocaprate, diglyceryl monolaurate or glyceryl monolaurate; polyhexamethylene biguanide salts, or mixtures thereof;
- zinc salts, for instance zinc salicylate, zinc phenolsulfonate, zinc pyrrolidonecarboxylate (more commonly known as zinc pidolate), zinc sulfate, zinc chloride, zinc lactate, zinc gluconate, zinc ricinoleate, zinc glycinate, zinc carbonate, zinc citrate, zinc chloride, zinc laurate, zinc oleate, zinc orthophosphate, zinc stearate, zinc silicate, zinc tartrate, zinc lactate and zinc acetate, or mixtures thereof.

In order to improve the antiperspirant efficacy of the composition, one or more water-soluble anionic polymers comprising a Brönsted acid, in particular those derived from maleic acid and/or from maleic anhydride, which are described in patent application WO 02/49590, may also be used.

In order to improve the homogeneity of the product, it is possible also to use one or more suspension agents preferably chosen from hydrophobic-modified montmorillonite clays, for instance hydrophobic-modified bentonites or hectorites. Examples that may be mentioned include the product stearalkonium bentonite (CTFA name) (product of reaction of bentonite and the quaternary ammonium stearalkonium chloride), such as the commercial product sold under the name Tixogel MP 250 by the company Süd Chemie Rheologicals, United Catalysts Inc. or the product disteardimonium hectorite (CTFA name) (product of reaction of hectorite and of distearyldimonium chloride) sold under the name Bentone 38 or Bentone Gel by the company Elementis Specialities.

The suspension agents are preferably present in amounts ranging from 0.1% to 5% by weight and more preferably from 0.2% to 2% by weight relative to the total weight of the composition.

The compositions according to the invention may also contain at least one organic powder.

Among the fillers that may be used according to the invention, mention may be made of organic powders. In the present patent application, the term "organic powder" means any solid that is insoluble in the medium at room temperature (25°C).

As organic powders that may be used in the composition of the invention, examples that may be mentioned include polyamide particles and especially those sold under the name Orgasol by the company Atochem; polyethylene powders; microspheres based on acrylic copolymers, such as those made of ethylene glycol dimethacrylate/lauryl methacrylate copolymer, sold by the company Dow Corning under the name Polytrap; polymethyl methacrylate microspheres, sold under the name Microsphere M-100 by the company Matsumoto or under the name Covabead LH85 by the company Wackherr; ethylene-acrylate copolymer powders, for instance those sold under the name Flobeads by the company Sumitomo Seika Chemicals; expanded powders such as hollow microspheres and especially microspheres formed from a terpolymer of vinylidene chloride, of acrylonitrile and of methacrylate and sold under the name Expancel by the company Kemanord Plast under the references 551 DE 12 (particle size of about 12 µm and density of 40 kg/m³), 551 DE 20 (particle size of about 30 µm and a density of 65 kg/m³) and 551 DE 50 (particle size of about 40 µm), or the microspheres sold under the name Micropearl F 80 ED by the company Matsumoto; powders of natural organic materials such as starch powders, especially of corn starch, wheat starch or rice starch, which may or may not be crosslinked, such as the starch powder crosslinked with octenylsuccinate anhydride, sold under the name Dry-Flo by the company National Starch; silicone resin microbeads such as those sold under the name Tospearl by the company Toshiba Silicone, especially Tospearl 240; amino acid powders such as the lauroyllysine powder sold under the name Amihope LL-11 by the company Ajinomoto; particles of wax microdispersion, which preferably have mean sizes of less than 1 µm and especially ranging from 0.02 µm to 1 µm, and which consist essentially of a wax or a mixture of waxes, such as the products sold under the name Aquacer by the company Byk Cera, and especially: Aquacer 520 (mixture of synthetic and natural waxes), Aquacer 514 or 513 (polyethylene wax), Aquacer 511 (polymer wax), or such as the products sold under the name Jonwax 120 by the company Johnson Polymer (mixture of polyethylene wax and paraffin wax) and under the name Ceraflour 961 by the company Byk Cera (micronized modified polyethylene wax); and mixtures thereof. The organic powder(s) may be present, for example, in an amount

The cosmetic compositions according to the invention may also comprise cosmetic adjuvants chosen from waxes, softeners, antioxidants, opacifiers, stabilizers, moisturizers, vitamins, fragrances, bactericides, preserving agents, polymers, fragrances, thickeners, propellants or any other ingredient usually used in cosmetics for this type of application.

Needless to say, a person skilled in the art will take care to select this or these optional additional compound(s) such that the advantageous properties intrinsically associated with the cosmetic composition in accordance with the invention are not, or are not substantially, adversely affected by the envisaged addition(s).

The waxes may be chosen from animal, fossil, plant, mineral and synthetic waxes. Mention may be made especially of beeswaxes, carnauba wax, candelilla wax, sugar cane wax, Japan wax, ozokerites, montan wax, microcrystalline waxes, paraffins and silicone waxes and resins.

The thickeners, which are preferably nonionic, may be chosen from modified or unmodified guar gums and celluloses, such as hydroxypropyl guar gum or cetylhydroxyethylcellulose, silicas, for instance Bentone Gel MIO sold by the company NL Industries, or Veegum Ultra sold by the company Polyplastic.

The amounts of these various constituents that may be present in the cosmetic composition according to the invention are those conventionally used in deodorant compositions.

The compositions according to the invention may also contain one or more other agents for structuring or gelling the water-immiscible organic liquid phase of the composition, such as linear solid fatty alcohols and/or waxes; fatty acids or salts thereof (stearic acid, sodium stearate or 12-hydroxystearic acid; dibenzylidene alditols (DBS); lanosterol, N-acylamino acid derivatives; di- or tricarboxylic acid derivatives, for instance alkyl-N,N'-dialkylsuccinamides (i.e.: dodecyl-N,N'-dibutylsuccinamide); elastomeric polyorganosiloxanes such as those described in patent application WO 97/44010.

The compositions according to the invention may also be pressurized and may be packaged in an aerosol device consisting of:
(A) a container comprising a deodorant composition as defined above,
(B) at least one propellant and a means for distributing the said aerosol composition.

The propellants generally used in products of this type, which are well known to those skilled in the art, are, for example, dimethyl ether (DME); volatile hydrocarbons such as n-butane, propane or isobutane, and mixtures thereof, optionally with at least one chlorohydrocarbon and/or fluorohydrocarbon; among the latter, mention may be made of the compounds sold by the company Dupont de Nemours under the names Freon^{®} and Dymel^{®}, and in particular monofluorotrichloromethane, difluorodichloromethane, tetrafluorodichloroethane and 1,1-difluoroethane sold especially under the trade name Dymel 152 A by the company Dupont. Carbon dioxide, nitrous oxide, nitrogen or compressed air may also be used as propellant.

The compositions containing the antiperspirant active polymer(s) as defined above and the propellant(s) may be in the same compartment or in different compartments in the aerosol container. According to the invention, the concentration of propellant generally ranges from 5% to 95% by pressurized weight and more preferably from 50% to 85% by weight relative to the total weight of the pressurized composition.

The distribution means, which forms a part of the aerosol device, generally consists of a distribution valve controlled by a distribution head, itself comprising a nozzle via which the aerosol composition is vaporized. The container containing the pressurized composition may be opaque or transparent. It may be made of glass, of polymeric material or of metal, optionally coated with a coat of protective varnish.

The examples that follow serve to illustrate the present invention.

### Example 1 : Antiperspirant emulsion (roll-on)

| **Phase** | **Ingredients** | **Amount in weight %** |
|---|---|---|
| A | Polyquaternium-7 (40%) (Salcare Super 7 - Ciba) | 20 |
| | Zinc gluconate (Givobio G Zn - SEPPIC) | 2 |
| B | Steareth-21 (Brij 721 - ICI) | 2 |
| | Steareth-2 (Brij 72 - ICI) | 2 |
| | Steareth-5 stearate (Arlatone 985 - ICI) | 1 |
| | PPG-15 stearyl ether (Arlamol E - ICI) | 1.5 |
| | Cyclopentasiloxane (DC 245 Fluid from Dow Corning) | 3.5 |
| C | Water | 68 |

### Procedure:

Phases (B) and (C) are separately heated to 70°C. (B) and (C) are mixed together with a Turrax blender for 5 minutes and then cooled to 55°C with paddle stirring. Phase A is added gently with stirring. The mixture is homogenized for 1 to 3 minutes. It is cooled to 35°C with stirring.

### Example 2 : Antiperspirant vaporizer (PIT emulsion)

| **Ingredients** | **Amount in weight %** |
|---|---|
| Polyquaternium-6 (40%) (Merquat 100 - Ondeo Nalco) | 20 |
| Cetearyl isononanoate (and) cetearyl alcohol (and) Ceteareth-20 (and) glycerol (and) glyceryl stearate (and) Ceteareth-12 (and) cetyl palmitate (Emulgade CM - Cognis) | 15 |
| Water | 65 |

### Procedure:

The polyquaternium-6 is dissolved in water and the "Emulgade CM" is added to the mixture with moderate stirring.

### Example 3 : Antiperspirant aerosol

| **Ingredients** | **Amount in weight %** |
|---|---|
| Stearalkonium bentonite (Tixogel MP 250 - Süd Chemie) | 0.5 |
| Polyquaternium-10 | 5 |
| (Ucare Polymer JR 125 - Amerchol) | |
| C₁₂₋₁₅ Alkyl benzoate (Finsolv TN - Witco) | 3 |
| Triethyl citrate (Citroflex 2: Morflex) | 1 |
| Isopropyl palmitate | 1 |
| Cyclopentasiloxane (DC 245 Fluid from Dow Corning) | 9.5 |
| Isobutane (propellant) | 80 |

## Claims

1. Use of a flocculating water-soluble polymer comprising amine groups borne by a side substituent directly attached to the main chain selected from :
(i) diallyldimethylammonium chloride (DADMAC) homopolymers with a molecular mass ranging from 10 000 to 1 000 000 g/mol ;
(ii) acrylamide/diallyldimethylammonium chloride copolymers ;
(iii) quaternary ammoniums of hydroxycellulose that have reacted with an epoxide substituted with a trimethylammonium ;
(iv) hydroxyalkyl celluloses grafted with a dimethyldiallylammonium salt ;
as an antiperspirant active agent in a cosmetic composition.

2. Use according to claim 1 of a flocculating water-soluble polymer in a cosmetic composition not containing any antiperspirant aluminium and/or zirconium salts.

3. Use according to any one of Claims 1 and 2, in which the flocculating water-soluble polymers have a molar mass ranging from 1000 to 20 000 000 g/mol and preferably ranging from 10 000 to 1 000 000 g/mol.

4. Use according to any one of Claims 1 to 3, in which the flocculating water-soluble polymer is present in amounts ranging from 0.1% to 50% and more preferably from 1% to 20% by weight relative to the total weight of the composition.

5. Use according to any one of Claims 1 to 4, in a composition that is in the form of a lotion, a cream or a fluid gel distributed as an aerosol spray, in a pump-dispenser bottle or as a roll-on; in the form of a cream or gel distributed in a tube or grille; in the form of a stick.

6. Use according to any one of Claims 1 to 5 , in an aerosol device consisting of:
(A) a container comprising a deodorant composition as defined according to any one of Claims 1 to 5;
(B) at least one propellant and a means for distributing the said aerosol composition.

7. Cosmetic process for treating human perspiration, which consists in applying to the surface of the skin an effective amount of a flocculating water-soluble polymer comprising amine groups borne by a side substituent directly attached to the main chain as defined according to any one of the preceding claims.

## Patentansprüche

1. Verwendung eines wasserlöslichen flockenden Polymers, das Aminogruppen aufweist, die von einem direkt an die Hauptkette gebundenen zeitlichen Substituenten getragen werden, ausgewählt unter:
(i) Diallyldiammoniumchlorid (DADMAC) Homopolymeren mit einer Molmasse von 10 000 bis 1 000 000 g/mol;
(ii) Acrylamid/Diallyldimethylammoniumchlorid-Copolymeren;
(iii) quartären Ammoniumverbindungen von Hydroxycellulose, die mit einem mit einer Trimethylammoniumgruppe substituierten Epoxid umgesetzt wurden;
(iv) Hydroxyalkylcellulosen, die mit einem Dimethyldiallylammoniumsalz gepfropft sind;
als schweißhemmender Wirkstoff in einer kosmetischen Zusammensetzung.

2. Verwendung eines wasserlöslichen flockenden Polymers nach Anspruch 1 in einer kosmetischen Zusammensetzung, die keine schweißhemmenden Aluminiumsalze und/oder Zirconiumsalze enthält.

3. Verwendung nach einem der Ansprüche 1 und 2, wobei die wasserlöslichen flockenden Polymere eine Molmasse von 1 000 bis 20 000 000 g/mol und vorzugsweise 10 000 bis 1 000 000 g/mol aufweisen.

4. Verwendung nach einem der Ansprüche 1 bis 3, wobei das wasserlösliche flockende Polymer in Mengenanteilen von 0,1 bis 50 Gew.-% und noch bevorzugter 1 bis 20 Gew.-%, bezogen auf das Gesamtgewicht der Zusammensetzung, enthalten ist.

5. Verwendung nach einem der Ansprüche 1 bis 4 in einer Zusammensetzung, die abgegeben als Aerosolspray, in einem Pumpflakon oder als Roll-on als Lotion, Creme oder fluides Gel; in Form einer Creme oder eines Gels, die aus einer Tube oder durch ein Gitter abgegeben werden; in Form eines Stiftes vorliegt.

6. Verwendung nach einem der Ansprüche 1 bis 5 in einer Aerosolvorrichtung, bestehend aus:
(A) einem Container, der eine Deodorantzusammensetzung enthält, wie sie in einem der Ansprüche 1 bis 5 definiert ist;
(B) mindestens einem Treibmittel und Einrichtungen für die Abgabe der Aerosolzusammensetzung.

7. Kosmetisches Verfahren für die Behandlung menschlicher Schweißabsonderung, das darin besteht, auf die Oberfläche der Haut eine wirksame Menge eines wasserlöslichen flockenden Polymers aufzubringen, das Aminogruppen aufweist, die von einem direkt an die Hauptkette gebundenen seitlichen Substituenten getragen werden, wie es nach einem der vorhergehenden Ansprüche definiert ist.

## Revendications

1. Utilisation d'un polymère hydrosoluble floculant comprenant des groupements amine portés par un substituant latéral lié directement à la chaîne principale, choisi parmi :
(i) les homopolymères de chlorure de diallyldiméthylammonium (DADMAC) ayant une masse moléculaire allant de 10 000 à 1 000 000 g/mole ;
(ii) les copolymères acrylamide/chlorure de diallyldiméthylammonium ;
(iii) les ammoniums quaternaires d'hydroxycellulose ayant réagis avec un époxyde substitué par un triméthylammonium ;
(iv) les hydroxyalkylcelluloses greffées avec un sel de diméthyldiallylammonium ;
en tant qu'agent antiperspirant actif dans une composition cosmétique.

2. Utilisation selon la revendication 1 d'un polymère hydrosoluble floculant dans une composition cosmétique ne contenant aucun sel d'aluminium et/ou de zirconium antiperspirant.

3. Utilisation selon l'une ou l'autre des revendications 1 et 2, **caractérisée en ce que** les polymères hydrosolubles floculants ont une masse molaire allant de 1000 à 20 000 000 g/mole et de préférence allant de 10 000 à 1 000 000 g/mole.

4. Utilisation selon l'une quelconque des revendications 1 à 3, **caractérisée en ce que** le polymère hydrosoluble floculant est présent dans des quantités allant de 0,1 % à 50 % et plus préférablement de 1 % à 20 % en poids par rapport au poids total de la composition.

5. Utilisation selon l'une quelconque des revendications 1 à 4, dans une composition qui est sous forme de lotion, de crème ou de gel fluide distribué en spray aérosol, dans un flacon pompe ou en roll-on ; sous forme de crème ou de gel distribué dans un tube ou une grille ; sous forme d'un bâtonnet.

6. Utilisation selon l'une quelconque des revendications 1 à 5, dans un dispositif aérosol constitué de :
(A) un récipient comprenant une composition déodorante telle que définie selon l'une quelconque des revendications 1 à 5 ;
(B) au moins un propulseur et un moyen pour distribuer ladite composition aérosol.

7. Procédé cosmétique de traitement de la transpiration humaine, **caractérisé en ce qu'**il consiste à appliquer à la surface de la peau une quantité efficace d'un polymère hydrosoluble floculant comprenant des groupements amine portés par un substituant latéral lié directement à la chaîne principale, tel que défini selon l'une quelconque des revendications précédentes.
